# EUROPÄISCHE PATENTSCHRIFT

(11) **EP 0 642 511 B1**
(45) Veröffentlichungstag und Bekanntmachung des Hinweises auf die Patenterteilung: **04.09.1996**
(21) Anmeldenummer: 93912713.0
(22) Anmeldetag: 18.05.1993
(51) Int. Cl.: C07D 487/04, A01N 43/90

(54) **VERFAHREN ZUR HERSTELLUNG VON 5-DICHLORACETYL-3,3,6-TRIMETHYL-9-OXO-1,5-DIAZABYCYCLO 4,3,0]NONAN**
METHOD OF PREPARING 5-DICHLOROACETYL-3,3,6-TRIMETHYL-9-OXO-1,5-DIAZABICYCLO 4,3,0]NONANE
PROCEDE DE FABRICATION DE 5-DICHLORACETYL-3,3,6-TRIMETHYL-9-OXO-1,5-DIAZABICYCLO 4,3,0]NONANE

(30) Priorität: 29.05.1992 DE 4217846
(43) Veröffentlichungstag der Anmeldung: 15.03.1995
(73) Patentinhaber: BASF AKTIENGESELLSCHAFT, 67056 Ludwigshafen (DE)
(72) Erfinder: REISSENWEBER, Gernot, D-6737 Boehl-Iggelheim (DE); RICHARZ, Winfried, D-6081 Stockstadt (DE); KOOB, Knut, D-6704 Mutterstadt (DE)
(86) Internationale Anmeldenummer: EP9301234
(87) Internationale Veröffentlichungsnummer: WO9324489

(56) Entgegenhaltungen:
- EP-A- 0 031 042
- EP-A- 0 190 105
- EP-A- 0 229 649
- HOUBEN-WEYL 'Methoden der Organischer Chemie, Bd. 11/2, 4. Auflage' 1958, GEORG THIEME VERLAG

## Beschreibung

Die vorliegende Erfindung betrifft ein verbessertes Verfahren zur Herstellung von 5-Dichloracetyl-3,3,6-trimethyl-9-oxo-1,5-diazabicyclo[4,3,0]nonan der Formel I 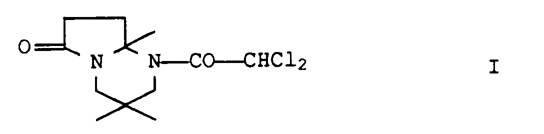 durch Umsetzung von 3,3,6-Trimethyl-9-oxo-1,5-diazabicyclo[4,3,0]nonan II 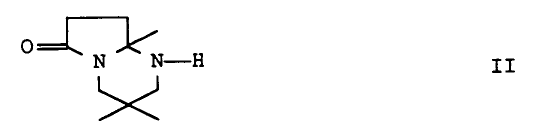 mit Dichloracetylchlorid in Gegenwart eines Lösungsmittels und einer Base.

Aus den EP-A 031 042, EP-A 190 105 und EP-A 229 649 ist bekannt, daß 5-Dichloracetyl-3,3,6-trimethyl-9-oxo-1,5-diazabicyclo[4,3,0]nonan (I) durch Umsetzung von 3,3,6-Trimethyl-9-oxo-1,5-diazabicyclo[4,3,0]nonan (II) mit Dichloracetylchlorid in Gegenwart eines Chlorwasserstoff bindenden Mittels erhalten werden kann, wobei als Lösungs- oder Verdünnungsmittel u.a. Toluol geeignet ist.

Gemäß der EP-A 229 649 kommen als Chlorwasserstoff bindende Mittel u.a. wäßrige Lösungen von Alkalimetallhydroxiden in Betracht. Im einzigen Ausführungsbeispiel der EP-A 031 042 und in Beispiel 4 aus der EP-A 229 649 ist jedoch nur die Reaktionsführung in Toluol mit Triethylamin als Chlorwasserstoff bindendem Mittel näher ausgeführt. Hierbei muß nach Beendigung der Reaktion erst festes Triethylaminhydrochlorid abgetrennt werden, wonach man (I) durch Eindampfen des Lösungsmittels, Abtrennen und anschließendes Umkristallisieren erhält. Diese Reaktionsführung mit zweimaliger Feststoffabtrennung (von Salz und Produkt) und anschließend getrennter Reinigung des Produktes ist verfahrenstechnisch und energetisch aufwendig.

Aufgabe der Erfindung war es daher, ein einfaches und technisch wirtschaftliches Verfahren zur Herstellung von I bereitzustellen, wobei I gleichzeitig in möglichst hoher Reinheit anfallen sollte.

Demgemäß wurde ein Verfahren zur Herstellung von 5-Dichloracetyl-3,3,6-trimethyl-9-oxo-1,5-diazabicyclo[4,3,0]-nonan I gefunden, welches dadurch gekennzeichnet ist, daß man
a) die Umsetzung von II mit Dichloracetylchlorid in einem Zweiphasensystem aus einem praktisch wasserunlöslichen organischen Lösungsmittel und Wasser vornimmt und hierbei Natrium- oder Kaliumhydroxid als Base nach Maßgabe des Verbrauches an Dichloracetylchlorid so zudosiert, daß die wässrige Phase einen pH-Wert von 7 bis 9 aufweist, und
b) das gebildete feste Produkt abtrennt.

Als organische Lösungsmittel eignen sich vornehmlich aromatische Kohlenwasserstoffe wie Benzol und vorzugsweise ein- oder mehrfach alkylierte Benzole mit 7 bis 12 C-Atomen wie Toluol, Ethylbenzol, Dimethylbenzole und Xylole.

Besonders bewährt hat sich ein 2-Phasensystem aus Toluol und Wasser.

Allgemein kommt ein Verhältnis von Wasser zu organischem Lösungsmittel zwischen 1 : 3 und 2 : 1 in Betracht, wobei die Menge an organischen Lösungsmittel bevorzugt zwischen 0,5 und 1,5 Litern pro Mol II liegt.

Das Natrium- oder Kaliumhydroxid, vorzugsweise Natriumhydroxid, wird in der Regel als wässrige Lösung eingesetzt.

Als besonders vorteilhaft hat es sich erwiesen, Dichloracetylchlorid und Natrium- oder Kaliumhydroxid synchron aber getrennt in das 2-Phasensystem aus organischem Lösungsmittel (enthaltend II) und Wasser einzubringen, wobei die Menge an Base so zu bemessen ist, daß der pH-Wert der wässrigen Phase während der gesamten Reaktion zwischen etwa 7 und 9, insbesondere zwischen 7,5 und 8,8, vorzugsweise zwischen 8,2 und 8,6, liegt. Nach bisherigen Erkenntnissen nimmt die Ausbeute an I rasch ab, je mehr der pH-Wert von 9 überschritten wird.

Um einen möglichst hohen Umsatz zu erzielen, setzt man Dichloracetylchlorid und II in etwa äquimolaren Mengen ein; vorteilhaft arbeitet man jedoch mit einem Überschuß an Dichloracetylchlorid bis etwa 50 mol-%, insbesondere mit einem Überschuß von 10 bis 20 mol-%, bezogen auf die Menge an II.

Im allgemeinen ist eine Reaktionstemperatur zwischen 0 und 80°C, insbesondere zwischen 20 und 60°C, bevorzugt zwischen 30 und 55°C, ausreichend.

Besondere Bedingungen bezüglich des Drucks sind nicht erforderlich; normalerweise nimmt man die Umsetzung daher bei Atmosphärendruck vor. Geringerer oder höherer Druck ist möglich, bringt aber normalerweise keine Vorteile.

Das während der Reaktion gebildete Produkt I scheidet sich als Feststoff aus dem Reaktionsgemisch aus. Es wird wie üblich abgetrennt, beispielsweise mittels Filtration oder Zentrifugieren.

Nach Waschen mit Wasser liegt das Produkt bereits in ausgezeichneter Reinheit vor, so daß sich weitere Reinigungsvorgänge in der Regel erübrigen.

Eine besondere vorteilhafte Ausführungsform des Verfahrens besteht darin, das Ausgangsprodukt II nach einem aus der DE-A 1 802 468 [vgl. auch Angew. Chem. 81, 34 (1969) sowie die dort zitierte Literatur] an sich bekannten Verfahren aus 4-Oxopentansäure und Neopentylamin herzustellen und ohne Isolierung aus der Reaktionsmischung im selben oder in einem anderen Reaktionsgefäß dem erfindungsgemäßen Verfahren zu unterwerfen.

Zweckmäßigerweise geht man dabei so vor, daß man 4-Oxopentansäure und Neopentylamin in dem gewünschten organischen Lösungsmittel unter laufender Entfernung des hierbei entstehenden Wassers, z.B. mittels azeotroper Destillation, reagieren läßt, bis kein Wasser mehr gebildet wird und der Siedepunkt der Reaktionsmischung konstanz bleibt. Durch Zufügen von Wasser oder dem während der Reaktion abgetrennten Wasser erhält man dann das für die weitere Umsetzung benötigte zweiphasige Lösungsmittelgemisch.

Die Menge an benötigtem Dichloracetylchlorid für die Überführung von II in I berechnet man bei dieser Ausführungsform zweckmäßigerweise nach der Menge an 4-Oxopentansäure anstelle von II.

Für einen vollständigen Umsatz an 4-Oxopentansäure und Neopentandiamin werden mindestens äquimolare Mengen oder ein geringer Überschuß der einen oder anderen Komponente, bis etwa 5 mol-%, benötigt. Größere Überschüsse eines Ausgangsstoffes sind auch möglich, bringen in der Regel aber keine Vorteile.

Die Menge an organischem Lösungsmittel ist so zu bemessen, daß sich die Ausgangsstoffe vollständig lösen. Normalerweise ist die 5- bis 10-fache Menge an Lösungsmittel, bezogen auf die Menge eines Eduktes, ausreichend.

Im allgemeinen liegt die Reaktionstemperatur für die Herstellung von II zwischen 40 und 140°C, vorzugsweise zwischen 60°C und der Siedetemperatur des Lösungsmittels.

Vorzugsweise arbeitet man bei Atmosphärendruck oder unter dem Eigendruck des jeweiligen Lösungsmittels.

Das erfindungsgemäße Verfahren stellt eine besondere Ausführungsform der Schotten-Baumann-Acylierung (vgl. z.B. Houben-Weyl, Methoden der Organischen Chemie, Bd. 11/2, 4. Auflage, Georg Thieme Verlag 1958, Seite 12 (13. Zeile von unten) und Seite 13) dar. Es ermöglicht die Herstellung von I auf einfache Weise in einer Reinheit von mehr als 97 %, wobei die Ausbeute sehr hoch ist.

5-Dichloracetyl-3,3,6-trimethyl-9-oxo-1,5-diazabicyclo[4,3,0]nonan I findet beispielsweise im Pflanzenschutz Verwendung, insbesondere als Antidot zur Verbesserung der Verträglichkeit von Herbiziden, z.B. aus der Klasse der Acetanilide, für Kulturpflanzen.

### Beispiele

### Vorstufe (Herstellung von 3,3,6-Trimethyl-9-oxo-1,5-diazabicyclo[4,3,0]nonan II)

119,6 g (1 mol) Lävulinsäure (97 %ig rein) in 600 ml Toluol wurden auf 105 bis 110°C erhitzt. Zu dieser Mischung tropfte man unter laufender destillativer Entfernung des entstehenden Reaktionswassers 103,0 g (1 mol) Neopentylamin (99 %ig rein), das durch Zusatz von 5 Gew.-% Toluol flüssig gehalten wurde. Nach fast vollständiger Zugabe des Neopentylamins wurde die Reaktionstemperatur auf ca. 115°C erhöht. Diese Temperatur wurde noch so lange beibehalten, bis die theoretische Menge an Wasser (= 36 g) abdestilliert war und die Übergangstemperatur des Destillats 110°C betrug. Danach wurde das Reaktionsgemisch auf etwa 50°C abgekühlt.

### Herstellung von I (erfindungsgemäß)

Das aus der Vorstufe erhaltene Reaktionsgemisch wurde mit 800 ml Wasser und dann mit 25 gew.%iger Natronlauge versetzt, bis die wässrige Phase einen pH-Wert von 8,4 hatte. In dieses 2-Phasengemisch tropfte man bei 40 bis 45°C innerhalb 1 Stunde 173,1 g (1,15 mol) Dichloracetylchlorid (98 %ig rein), wobei der pH-Wert der wässrigen Phase durch laufende Zugabe von 25 gew.%iger Natronlauge (insgesamt 216,0 g) konstant gehalten wurde. Schließlich wurde das Produktgemisch noch 30 Minuten gerührt und dann auf 20 bis 25°C abgekühlt. Das feste Produkt wurde abgetrennt und mit ca. 500 ml Wasser gewaschen. Fp.: 171°C
Ausbeute: 92,7 % mit einer Reinheit von 98,4 % (nach HPLC-Analyse).

### Vergleichsbeispiel A

Zu dem aus der Vorstufe erhaltenen Reaktionsgemisch wurden bei 40 bis 45°C innerhalb einer Stunde 173,1 g (1,15 mol) Dichloracetylchlorid (98 %ig rein) und 216,0 g einer 25 gew.-%igen Natronlauge gegeben.

Schließlich wurde das Produktgemisch noch 30 Minuten gerührt und dann auf 20 bis 25°C abgekühlt. Das feste Produkt wurde abgetrennt und mit ca. 500 ml Wasser gewaschen.
Ausbeute: 81,8 % mit einer Reinheit von 89,9 % (nach HPLC-Analyse).

### Vergleichsbeispiel B

Zu dem aus der Vorstufe erhaltenen Reaktionsgemisch wurden zuerst 216,0 g einer 25 gew.%igen Natronlauge gegeben. In dieses Gemisch tropfte man bei 40 bis 45°C innerhalb 1 Stunde 173,1 g (1,15 mol) Dichloracetylchlorid (98 %ig rein).

Schließlich wurde das Produktgemisch noch 30 Minuten gerührt und dann auf 20 bis 25°C abgekühlt. Das feste Produkt wurde abgetrennt und mit ca. 500 ml Wasser gewaschen.
Ausbeute: 82,9 % mit einer Reinheit von 94,1 % (nach HPLC-Analyse).

## Patentansprüche

1. Verfahren zur Herstellung von 5-Dichloracetyl-3,3,6-trimethyl-9-oxo-1,5-diazabicyclo[4,3,0]-nonan (I) 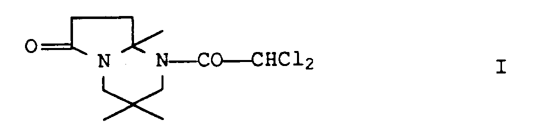 durch Umsetzung von 3,3,6-Trimethyl-9-oxo-1,5-diazabicyclo[4,3,0]nonan II 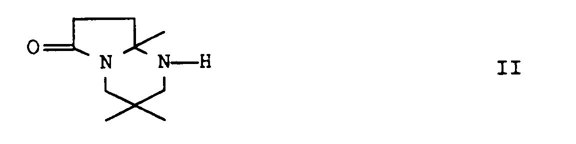 mit Dichloracetylchlorid in Gegenwart eines Lösungsmittels und einer Base, dadurch gekennzeichnet, daß man
a) diese Umsetzung in einem Zweiphasensystem aus einem praktisch wasserunlöslichen organischen Lösungsmittel und Wasser vornimmt und hierbei Natrium- oder Kaliumhydroxid als Base nach Maßgabe des Verbrauches an Dichloracetylchlorid so zudosiert, daß die wässrige Phase einen pH-Wert von 7 bis 9 aufweist, und
b) das gebildete feste Produkt abtrennt.

2. Verfahren nach Anspruch 1, dadurch gekennzeichnet, daß man von einer Mischung von 3,3,6-Trimethyl-9-oxo-1,5-diazabicyclo[4,3,0]nonan II in einem praktisch wasserunlöslichen organischen Lösungsmittel ausgeht, wie sie bei der Herstellung von II aus 4-Oxopentansäure und Neopentandiamin unter laufender Entfernung des hierbei entstehenden Wassers anfällt.

3. Verfahren nach Anspruch 1 oder 2, dadurch gekennzeichnet, daß man als organisches Lösungsmittel ein ein- oder mehrfach alkyliertes Benzol mit 7 bis 12 C-Atomen verwendet.

4. Verfahren nach Anspruch 1 oder 2, dadurch gekennzeichnet, daß man die Umsetzung bei einem pH-Wert von 7,5 bis 8,8 vornimmt.

5. Verfahren nach Anspruch 1 oder 2, dadurch gekennzeichnet, daß man die Reaktion bei einer Temperatur zwischen 20 und 60°C vornimmt.

## Claims

1. A process for preparing 5-dichloroacetyl-3,3,6-trimethyl-9-oxo-1,5-diazabicyclo[4,3,0]nonane (I) 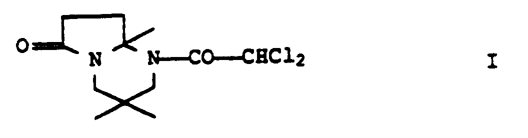 by reacting 3,3,6-trimethyl-9-oxo-1,5-diazabicyclo[4,3,0]nonane II 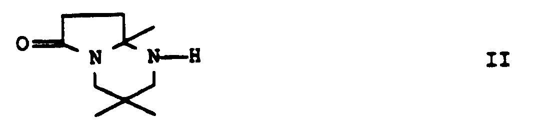 with dichloroacetyl chloride in the presence of a solvent and of a base, wherein
a) this reaction is carried out in a two-phase system consisting of a virtually water-insoluble organic solvent and water and, in this case, sodium or potassium hydroxide is metered in as base in accordance with the consumption of dichloroacetyl chloride in such a way that the aqueous phase has a pH of from 7 to 9, and
b) the solid product which is formed is separated off.

2. A process as claimed in claim 1, which starts from a mixture of 3,3,6-trimethyl-9-oxo-1,5-diazabicyclo[4,3,0]nonane II in a virtually water-insoluble organic solvent as is produced in the preparation of II from 4-oxopentanoic acid and neopentanediamine with continuous removal of the water produced thereby.

3. A process as claimed in claim 1 or 2, wherein a mono- or polyalkylated benzene with 7 to 12 carbon atoms is used as organic solvent.

4. A process as claimed in claim 1 or 2, wherein the reaction is carried out at a pH of from 7.5 to 8.8.

5. A process as claimed in claim 1 or 2, wherein the reaction is carried out at from 20 to 60°C.

## Revendications

1. Procédé de préparation du 5-dichloracétyl-3,3,6-triméthyl-9-oxo-1,5-diazabicyclo[4,3,0]nonane (I) 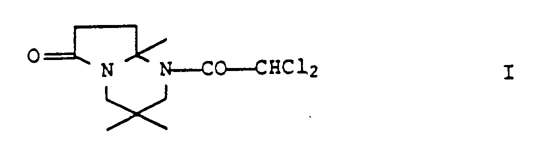 par réaction du 3,3,6-triméthyl-9-oxo-1,5-diazabicyclo[4,3,0]nonane (II) 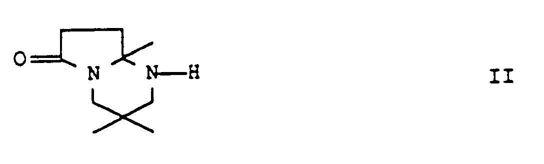 avec le chlorure de dichloracétyle en présence d'un solvant et d'une base, caractérisé par le fait que
a) on procède à cette réaction dans un système à deux phases consistant en un solvant organique pratiquement insoluble dans l'eau et de l'eau et on ajoute, en tant que base, de l'hydroxyde de sodium ou de potassium au fur et à mesure de la consommation du chlorure de dichloracétyle, en sorte que la phase aqueuse ait un pH de 7 à 9, et
b) on sépare le produit solide formé.

2. Procédé selon la revendication 1, caractérisé par le fait que l'on part d'un mélange du 3,3,6-triméthyl-9-oxo-1,5-diazabicyclo[4,3,0]nonane II dans un solvant organique pratiquement insoluble dans l'eau, tel qu'obtenu à la préparation de II à partir de l'acide 4-oxo-pentanoïque et de la néopentanediamine avec élimination en continu de l'eau formée dans la réaction.

3. Procédé selon la revendication 1 ou 2, caractérisé par le fait que l'on utilise en tant que solvant organique un benzène mono- ou polyalkylé en C7-C12.

4. Procédé selon la revendication 1 ou 2, caractérisé par le fait que l'on effectue la réaction à un pH de 7,5 à 8,8.

5. Procédé selon la revendication 1 ou 2, caractérisé par le fait que l'on effectue la réaction à une température de 20 à 60°C.
